Europäisches Patentamt

European Patent Office

Office européen des brevets

(19)

(11) Veröffentlichungsnummer: **0 008 454**
**B1**

(12) **EUROPÄISCHE PATENTSCHRIFT**

(45) Veröffentlichungstag der Patentschrift: **28.04.82**

(51) Int. Cl.³: **A 61 L 2/02**

(21) Anmeldenummer: **79103046.3**

(22) Anmeldetag: **20.08.79**

(54) **Verfahren zur Sterilisierung von pharmazeutischen Wirkstoffen.**

(30) Priorität: **25.08.78 DE 2837115**

(43) Veröffentlichungstag der Anmeldung:
**05.03.80 Patentblatt 80/5**

(45) Bekanntmachung des Hinweises auf die Patenterteilung:
**28.04.82 Patentblatt 82/17**

(84) Benannte Vertragsstaaten:
**BE CH DE FR GB IT NL SE**

(56) Entgegenhaltungen:
**GB - A - 1 188 753**

(73) Patentinhaber: **BAYER AG**
**Zentralbereich Patente, Marken und Lizenzen**
**D-5090 Leverkusen 1, Bayerwerk (DE)**

(72) Erfinder: **Pilz, Volker, Dr.**
**Selbacherweg 7**
**D-5000 Köln 91 (DE)**
Erfinder: **Rupp, Roland, Dr.**
**Karl-Arnold-Strasse 9**
**D-5090 Leverkusen (DE)**

Courier Press, Leamington Spa, England.

Verfahren zur Sterilisierung von pharmazeutischen Wirkstoffen

Die vorliegende Erfindung betrifft ein Verfahren zur Sterilisierung von pharmazeutischen Wirkstoffen, durch Sterilfiltration unter Einsatz von überkritischen Gasen.

Für die Herstellung steriler Pulver und Feststoffe sind bisher eine Reihe von Sterilisationsverfahren bekannt. Bei thermisch stabilen Verbindungen wird häufig die Hitzesterilisation, d.h. eine Erwärmung auf 140 bis 180°C verwendet. Bei Temperaturen unter 140°C ist eine vollständige Sterilisation oft nicht gewährleistet. Die Pulversterilisation durch Anwendung antiseptischer Gase, insbesondere durch Begasung mit Äthylenoxid, ist technisch nur unter besonderen Vorsichtsmaßnahmen durchzuführen, da Äthylenoxid mit Luft ein explodierbares Gemisch bildet. Darüber hinaus werden durch solche Begasungsverfahren ebenso durch die Hitzesterilisation keine weiteren Reinigungseffekte, wie z.B. Abtrennung von Fasern und anderen festen Fremdpartikeln erreicht. Bei besonders reaktionsfähigen Substanzen besteht außerdem die Gefahr einer chemischen Reaktion mit Äthylenoxid (vgl. Galenisches Praktikum, K. Münzel et al, 822—829, (1959) und Hagers Handbuch der Pharmazeutischen Praxis, 4. Ausgabe, Bd. 7, Teil A, 369 (1971)).

Weiterhin sind Verfahren zur Herstellung von sterilen Pulvern mittels Gefriertrocknung von sterilfiltrierten, meist wäßrigen Arzneistofflösungen bekannt. Diese Methoden sind technisch aufwendig und nur anwendbar bei Substanzen, die wasserlöslich und hydrolysebeständig sind.

Sterilherstelllung von Pulvern unter Verwendung organischer Lösungsmittel ist ebenfalls gebräuchlich, wobei die gelöste Substanz zunächst durch einen Sterilfilter geschickt wird und anschließend aus dem Lösungsmittel ausgefällt wird. Ein wesentlicher Nachteil dieses Verfahrens liegt darin, daß die so aufgearbeiteten Substanzen noch Lösungsmittelrückstände enthalten und nur durch aufwendige Methoden lösungsmittelfrei erhalten werden können.

Der erwähnte Nachteil bei der Sterilherstellung kann vermieden werden, wenn man statt flüssiger, organischer Lösemittel andere Stoffe benutzt, die einerseits in der Lage sind, die zu sterilisierenden Substanzen zu lösen, die sich andererseits aber selbst kaum in diesen Substanzen lösen, so daß eine quantitative Rückgewinnung des Gelösten einfach und leicht möglich ist.

Hier bieten sich Gase an, von denen bekannt ist, daß sie im verdichteten und insbesondere im überkritischen Zustand in der Lage sind, auch schwerflüchtige Substanzen zu lösen. Bei Druckabsenkung geht diese Lösefähigkeit sehr rasch (häufig schon in der Gegend von 20 bis 50 bar) verloren und auch die Löslichkeit des Gases in der dann ausfallenden Substanz wird mit fallendem Druck so gering, daß bei Druckabsenkung auf Umgebungsdruck vorher gelöste Stoffe sehr rein zurückgewonnen werden.

Der beschriebene Löslichkeitseffekt in verdichteten überkritischen Gasen, der übrigens schon seit nahezu 100 Jahren bekannt ist (s. z.B. Hannay und Hogarth, Proc. Roy. Soc. 30 (1980), 178), zwischendurch aber wieder in Vergessenheit geriet, wird in neuester Zeit bei der Auftrennung von Stoffgemischen ausgenutzt. So wurden in der Deutschen Patentschrift 2 005 293 ein Verfahren zur Entcoffeinierung von Rohkaffee und in der Patentschrift 2 043 537 ein Verfahren zur Extraktion von Nikotin aus Tabak mit überkritischen Gasen (z.B. Kohlendioxid) beschrieben. Verfahren zur Herstellung von Hopfen- bzw. Gewürzextrakten, ebenfalls unter Verwendung überkritischer Gase, sind in den Patentschriften 2 127 618 und 2 127 611 beschrieben. Außerdem wird has Lösevermögen überkritischer Gase eingesetzt in der Hochdruckgaschromatographie (s. z.B. Chemie-Ingenieur-Technik, 42. Jahrgang, Heft 13 (1970), SS. 702/704 und 890/898).

Die Verwendung von überkritischen Gasen zur Sterilherstellung von Feststoffen, insbesondere von pharmazeutischen Wirkstoffen ist bisher nicht bekannt geworden.

Die vorliegende Erfindung betrifft ein Verfahren zur Sterilisierung von pharmazeutischen Wirkstoffen, dadurch gekennzeichnet, daß der Wirkstoff unter überkritischen Bedingungen bezüglich Druck und Temperatur eines Gases oder Gasgemisches von diesem gelöst und durch einen Sterilfilter transportiert wird.

Dieses Sterilisierungsverfahren mit überkritischen Gasen ist besonders geeignet für Festoffe, an die höchste Reinheitsforderungen gestellt werden, insbesondere für Arzneimittelwirkstoffe. Das erfindungsgemäße Verfahren erlaubt eine diskontinuierliche oder eine kontinuierliche Arbeitsweise, wobei unter schonenden Bedingungen empfindliche Arzneimittelwirkstoffe verlustfrei und ohne störende Lösungsmittel-Verunreinigungen steril erhalten werden können. Als Arbeitsgas für die erfindungsgemäße Sterilfiltration kommen in Betracht: Kohlendioxid, Distickstoffoxid, niedere Kohlenwasserstoffe, (z.B. Äthan, Äthylen, Propan, Propylen), niedere Halogenkohlenwasserstoffe (z.B. Trifluorchlormethan, Fluoroform $(CHF_3)$, Difluorchlormethan) sowie Schwefelhexafluorid und Ammoniak und zwar allein oder in Mischungen untereinander sowie "dotiert", d.h. versehen mit kleinen Prozentsätzen an mittel- und höhersiedenden Komponenten, wie z.B. Wasser, Alkoholen und höheren Kohlenwasserstoffen.

Das erfindungsgemäße Verfahren wird zweckmäßigerweise in einem Temperaturbereich von 20 bis 140°C durchgeführt.

Vorzugsweise arbeitet man bei einem Temperaturbereich von 30 bis 80°C.

Das Verfahren wird normalerweise bei Drücken zwischen 20 und 1200 bar durchgeführt. Vorzugsweise arbeitet man bei Drücken zwischen 50 und 1000 bar.

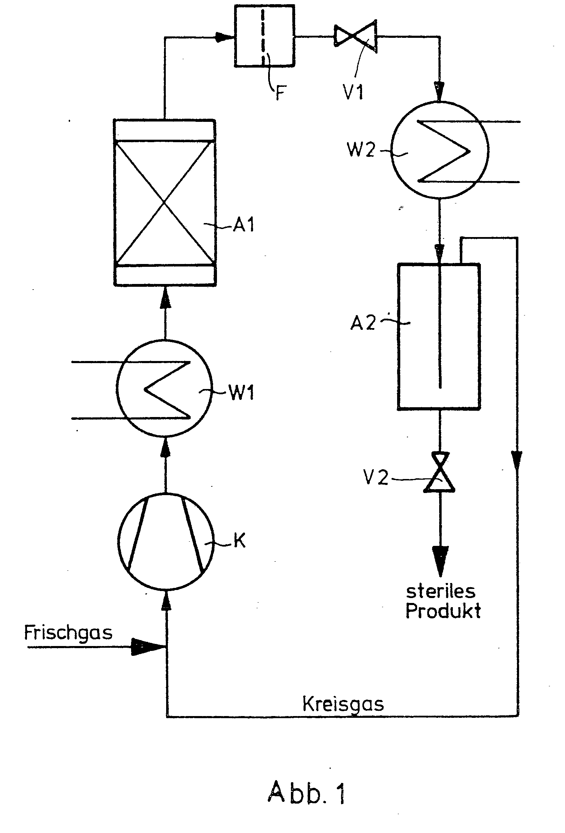

Eine mögliche technische Ausführungsform des Verfahrens sei anhand der Abbildung 1 erläutert:

Das Arbeitsgas wird in einem Kompressor K auf den erwünschten Arbeitsdruck verdichtet und in nachgeschalteten Wärmetauscher W1 auf eine Temperatur gebracht, die in der Regel nur einige Grad über der kritischen Temperatur des Gases (oder Gasgemisches) liegt. Im Apparat A1, einem Lösungsbehälter, in dem das keimfrei zu machende Produkt vorgelegt ist, wird das Gas nun mit diesem Produkt beladen. Anschließend wird das sich bildende fluide Gemisch durch das Sterilfilter F geschickt, wo Bakterien und Keime zurückgehalten werden. Das sterile, homogene Gas/Substanz-Gemisch wird dann durch En Spannung am Ventil V1 und/oder Temperaturänderung im Wärmetauscher W2 in zwei Phasen aufgetrennt, nämlich die sterile Feststoffphase, die als steriles Endprodukt unten am Druckbehälter (Abscheider) A2 abgenommen werden kann, und in eine geringer verdichtete, vom Feststoff weitestgehend befreite Gasphase, die als Kreisgas zurück zum Kompressor geführt wird.

Weitere Ausführungsformen dieses Verfahrens sind dadurch gekennzeichnet, daß, das Arbeitsgas nach der Verdichtung mit höhersiedenden Komponenten (Wasser, Alkohole, Kohlenwasserstoffe) dotiert werden kann, daß Löservorgang und insbesondere Abscheidevorgang in mehreren Stufen, die sich durch unterschiedlichen Druck und/oder unterschiedliche Temperatur auszeichnen, erfolgen und dadurch, daß das Verfahren auch im Hinblick auf das zu sterilisierende Produkt kontinuierlich betrieben wird, d.h. mit stetiger Zu- und Abfuhr von Substanz.

Beispiel 1

In einer Versuchsapparatur nach Abbildung 1 war in einem Gefäß A1 eine bestimmte Menge Acetylsalicylsäure in Form einer Pulverschüttung vorgelegt. Diese Schüttung wurde kontinuierlich von überkritischem Kohlendioxid von einer Temperatur von T=50°C durchströmt. Die auf den freien Querschnitt bezogene Gasgeschwindigkeit lag dabei in der Größenordnung von $w_o=0,1\div1\cdot10^{-3}$ m/sec, die Kontaktzeit zwischen Gas und Feststoff bei 5 bis 30 Minuten. Bei einem Druck von 500 bar im Behälter A1 und von 1 bis 50 bar im Behälter A2 fielen dabei je Normkubikmeter umgepumptes Kohlendioxid 1,9 g steriles Produkt an.

Beispiel 2

Bei gleichen Bedingungen wie in Beispiel 1, allerdings mit einem Lösedruck von P=900 bar, war die Ausbeute: 4,9 g steriles Produkt je Normkubikmeter umgepumptes Kohlendioxid.

Beispiel 3

In der gleichen Anordnung wie in Beispiel 1 wurde der Wirkstoff Ampicillin-Natrium mit $N_2O$ bei 40°C und 100 bar sterilisiert. Je Normkubikmeter $N_2O$ fielen 0,1 g steriler Wirkstoff an.

Beispiel 4

Unter gleichen Bedingungen wie in Beispiel 3 fielen vom Wirkstoff Azlocillin-Natrium 0,15 g sterile Substanz je Normkubikmeter $N_2O$ an.

Beispiel 5

Bei 100 bar und 30°C fielen unter Verwendung von Frigen 13, dotiert mit 2% Frigen 11, in der Anordnung nach Beispiel 1 70 mg je Normkubikmeter Gasgemisch des Wirkstoffes Azlocillin-Natrium an.

**Patentansprüche**

1. Verfahren zur Sterilisierung von pharmazeutischen Wirkstoffen, dadurch gekennzeichnet, daß der Wirkstoff unter überkritischen Bedingungen bezüglich Druck und Temperatur eines Gases oder Gasgemisches von diesem gelöst und durch einen Sterilfilter transportiert wird.

2. Verfahren gemäß Anspruch 1, dadurch gekennzeichnet, daß bei einem Temperaturbereich von 20 bis 140°C und in einem Druckbereich von 20 bis 1200 bar gearbeitet wird.

3. Verfahren gemäß Anspruch 1, dadurch gekennzeichnet, daß man in einem Temperaturbereich von 30 bis 80°C und in einem Druckbereich von 50 bis 1000 bar arbeitet.

4. Verfahren gemäß Anspruch 1, dadurch gekennzeichnet, daß das sterile, fluide Gas/Feststoff-Gemisch nach dem Filter durch Druckentspannung und/oder Temperaturvariation wieder in Feststoff und Gas getrennt wird.

5. Verfahren gemäß Anspruch 1 bis 4, dadurch gekennzeichnet, daß das Arbeitsgas ganz oder teilweise in einem Kreislauf geführt wird.

**Claims**

1. Process for the sterilisation of pharmaceutical active ingredients, characterised in that, under supercritical conditions, with respect to pressure and temperature, of a gas or gas mixture, the active ingredient is dissolved by the latter and transported through a sterile filter.

2. Process according to Claim 1, characterised in that it is carried out in a temperature range from 20 to 140°C and in a pressure range from 20 to 1,200 bars.

3. Process according to Claim 1, characterised in that it is carried out in a temperature range from 30 to 80°C and in a pressure range from 50 to 1,000 bars.

4. Process according to Claim 1, characterised in that the sterile fluid gas/solid mixture is separated into solid and gas again, downstream from the filter, by letting down the pressure and/or varying the temperature.

5. Process according to Claim 1 to 4, characterised in that all or part of the working gas is circulated.

## Revendications

1. Procédé pour stériliser des substances actives pharmaceutiques, caractérisé en ce que la substance active est dissoute dans un gaz ou un mélange gazeux dans des conditions de température et de pression supérieures aux conditions critiques et transportée au travers d'un filtre stérilisant.

2. Procédé selon la revendication 1, caractérisé en ce que l'on opère dans un intervalle de température de 20 à 140°C et un intervalle de pression de 20 à 1200 bars.

3. Procédé selon la revendication 1, caractérisé en ce que l'on opère dans un intervalle de température de 30 à 80°C et dans un intervalle de pression de 50 à 1000 bars.

4. Procédé selon la revendication 1, caractérisé en ce que le mélange fluide et stérile gaz/substance solide est à nouveau séparé en gaz et substance solide après le filtre par détente de pression et/ou variation de température.

5. Procédé selon les revendications 1 à 4, caractérisé en ce que le gaz servant aux opérations est recyclé en totalité ou en partie.

Abb. 1

1